# EUROPEAN PATENT APPLICATION

(11) **EP 2 749 263 A1**
(43) Date of publication of application: **02.07.2014**
(21) Application number: 12827722.5
(22) Date of filing: 21.08.2012
(51) Int. Cl.: A61J 3/00, G06Q 50/24

(54) **SEPARATE PACKAGING DEVICE FOR MEDICINE**

(30) Priority: 26.08.2011 JP 2011184743
(71) Applicant: Takazono Technology Incorporated, Osaka 573-0128 (JP)
(72) Inventor: IIMORI, Kazuo, Hirakata-shi Osaka 573-0128 (JP); TAKAMATSU, Akira, Hirakata-shi Osaka 573-0128 (JP)
(74) Representative: Grünecker, Kinkeldey, Stockmair & Schwanhäusser
(86) International application number: PCT/JP2012/071076
(87) International publication number: WO 2013/031581

(57) **Abstract**

A packaging device eliminating waste of package paper can be provided. The packaging device includes: a computer (20) that creates one packaging data based on prescription data regarding a plurality of patients belonging to the same group; and a packaging machine (100) that packages a drug based on the packaging data created by the computer (20).

## Description

### TECHNICAL FIELD

The present invention relates to a drug packaging device that packages a drug.

### BACKGROUND ART

A conventional drug packaging device is disclosed in, for example, Japanese Patent Laying-Open No. 61-232113 (Patent Document 1).

### CITATION LIST

### PATENT DOCUMENT

PTD 1: Japanese Patent Laying-Open No. 61-232113

### SUMMARY OF INVENTION

### TECHNICAL PROBLEM

In the drug packaging device of Patent Document 1, a series of package bags are formed for each patient. On each package bag, the name of the patient, dosing time, and the like are printed. A printer, which is a printing unit for printing the name of the patient, the dosing time, and the like on each package bag, is provided upstream of a location via which a drug is introduced to package paper.

In this technique, an empty bag having no drug contained therein is formed between package bags for patients due to its structure. This leads to waste of the package paper. In particular, when the number of packages is small with respect to the number of patients, the package paper is greatly wasted, disadvantageously. Accordingly, the present invention has been made to solve the foregoing problem, and has an object to provide a drug packaging device capable of reducing waste of package paper.

### SOLUTION TO PROBLEM

A drug packaging device according to the present invention include: a packaging data creating unit that creates one piece of packaging data based on prescription data regarding a plurality of patients belonging to a same group; and a drug packaging unit that packages a drug based on the packaging data created by the packaging data creating unit.

In the drug packaging device thus configured, the one piece of packaging data is created based on the prescription data regarding the plurality of patients belonging to the same group. Based on this one piece of packaging data, the drug is packaged, whereby no empty package is formed between package bags for different patients of the same group. Accordingly, waste of the package paper can be reduced.

Preferably, the packaging data creating unit obtains the prescription data regarding the plurality of patients belonging to the same group, from a host computer as one file.

Preferably, the packaging data is an aggregate of pieces of data each for one package, and each of the pieces of data each for one package includes data regarding a packaging speed.

Preferably, the packaging data is an aggregate of pieces of data each for one package, and each of the pieces of data each for one package includes data regarding a package size.

Preferably, the drug packaging unit has a manually prepared drug supplying unit that sequentially supplies drugs contained in a plurality of compartments formed in a tray, and is configured to package the drugs supplied from the manually prepared drug supplying unit. The drug packaging device further includes: a manual preparation instruction data creating unit that extracts data regarding a manually prepared drug from the prescription data regarding the plurality of patients belonging to the same group, and that creates manual preparation instruction data designating a compartment, which is to contain the manually prepared drug, of the tray; and a manual preparation instruction data output unit that outputs the manual preparation instruction data created by the manual preparation instruction data creating unit.

### BRIEF DESCRIPTION OF DRAWINGS

Fig. 1 is a block diagram of a drug preparing system having a drug packaging device according to a first embodiment of the present invention.
Fig. 2 is a perspective view showing an internal configuration of a packaging machine.
Fig. 3 is a plan view of a tray attached to the packaging machine shown in Fig. 2.
Fig. 4 is a flowchart for illustrating an operation regarding preparation of prescription data in a host computer.
Fig. 5 is a flowchart for illustrating an operation regarding obtainment of the prescription data in a computer.
Fig. 6 is a flowchart for illustrating an operation regarding creation of packaging data in the computer.
Fig. 7 illustrates the packaging data.
Fig. 8 is a flowchart for illustrating an operation regarding creation of manual preparation instruction data in the computer.
Fig. 9 shows contents of a manual preparation instruction.
Fig. 10 illustrates a result of packaging.

### DESCRIPTION OF EMBODIMENTS

The following describes embodiments of the present invention with reference to figures. It should be noted that in the below-mentioned embodiments, the same or corresponding portions are given the same reference characters and are not described repeatedly. In addition, the embodiments can be combined with each other.

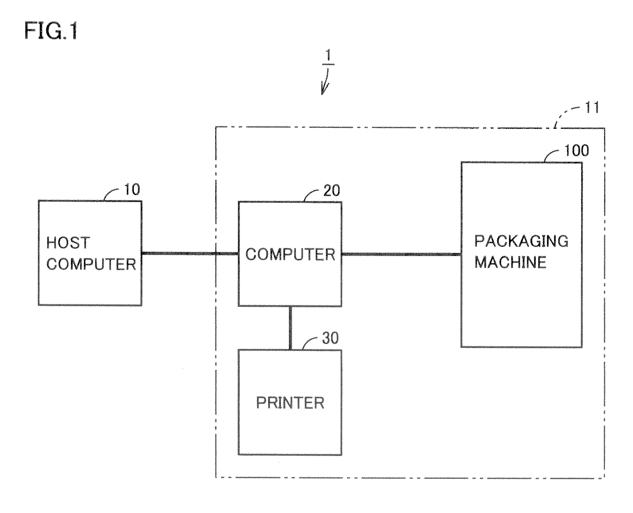

Fig. 1 is a block diagram of a drug preparing system having a drug packaging device according to a first embodiment of the present invention. Referring to Fig. 1, drug preparing system 1 includes a host computer 10, and a drug packaging device 11 serving as a drug preparation device.

Host computer 10 creates, as one file, prescription data regarding a plurality of patients belonging to the same group. In other words, based on the group as a unit, host computer 10 collectively creates the prescription data regarding the plurality of patients as one piece of prescription data. In this way, the prescription data regarding the plurality of patients belonging to the same group is handled as one piece of prescription data, thereby simplifying management of prescription data. The group may be made based on a hospital ward as a unit, or based on a medical department as a unit. The group may be set based on an appropriate unit.

The prescription data includes: group data regarding the group; patient data regarding the patients; dosing time data regarding dosing time; drug data regarding a drug. The group data is associated with a plurality of pieces of patient data. Each piece of patient data is associated with one or a plurality of pieces of dosing time data. Each piece of dosing time data is associated with one or a plurality of pieces of drug data.

Drug packaging device 11 includes: a computer 20 connected to host computer 10; a packaging machine 100 connected to computer 20 and packaging a drug based on packaging data sent from computer 20; and a printer 30 connected to computer 20 and printing a manual preparation instruction based on manual preparation instruction data sent from computer 20. Packaging machine 100 serves as a drug packaging unit. Printer 30 serves as a manual preparation instruction data output unit.

From host computer 10, computer 20 obtains, as one file, prescription data regarding a plurality of patients belonging to the same group. As compared with a case where pieces of prescription data regarding patients are sequentially obtained, a communication process can be simplified. Based on the prescription data obtained from host computer 10, computer 20 creates packaging data, which is drug preparation data, and creates manual preparation instruction data. Thus, computer 20 serves as a packaging data creating unit, and also serves as a manual preparation instruction data creating unit.

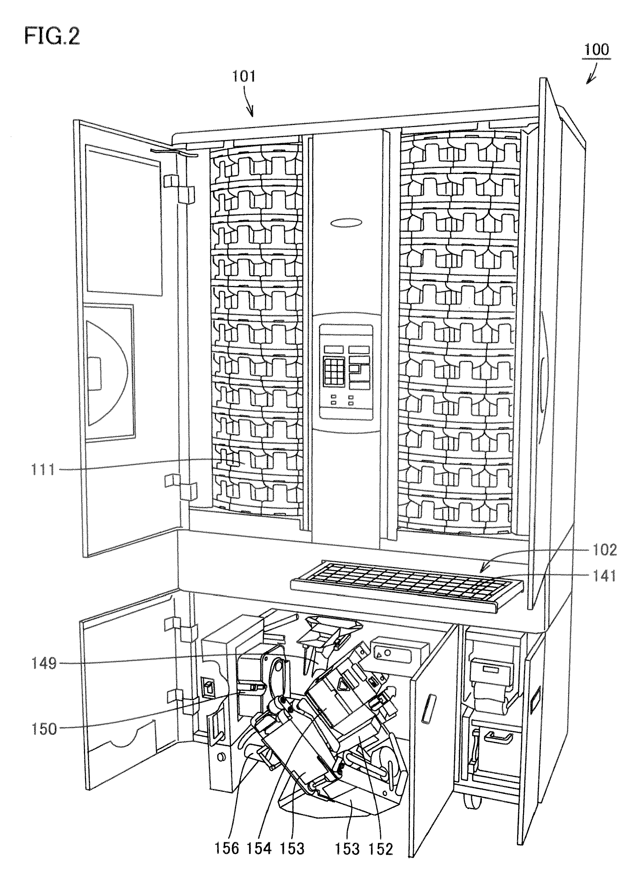

Fig. 2 is a perspective view showing an internal configuration of the packaging machine. Referring to Fig. 2, packaging machine 100, which is a drug preparing machine, includes a cassette drug supplying unit 101 and a manually prepared drug supplying unit 102, and is configured to package drugs supplied from cassette drug supplying unit 101 and manually prepared drug supplying unit 102. Cassette drug supplying unit 101 has a plurality of cassettes 111 having drugs contained therein, and selectively supplies the drugs contained in cassettes 111. Cassettes 111 are vertically arranged in multiple stages. Manually prepared drug supplying unit 102 has a tray 141 having a plurality of compartments 141a formed therein, and sequentially supplies drugs contained in compartments 141 a. Manually prepared drug supplying unit 102 is used to supply drugs that cannot be supplied from cassette drug supplying unit 101.

Provided below cassette drug supplying unit 101 and manually prepared drug supplying unit 102 are: a package paper roll supporting unit 156 supporting a package paper roll formed by rolling package paper; a sealing unit 154 forming a series of package bags by performing heat sealing while sending the package paper withdrawn from the package paper roll supported by package paper roll supporting unit 156; a hopper 149 disposed upstream of sealing unit 154 and introducing drugs, which are supplied from cassette drug supplying unit 101 and manually prepared drug supplying unit 102, to the package paper; a cutting unit 152 disposed downstream of sealing unit 154 and cutting the package paper; and a transporting unit 153 disposed downstream of cutting unit 152 and transporting the package paper.

A printing unit 150 is provided upstream of hopper 149 so as to print information regarding a drug onto the package paper. Printing unit 150 prints the information regarding the drug onto the package paper before the heat sealing of the package paper by sealing unit 154 such that the information regarding the drug contained in the package bag formed by sealing unit 154 is clearly indicated on the package bag. The information regarding the drug includes: the name of the patient, the dosing time, the name of the drug, the quantity thereof, and the like.

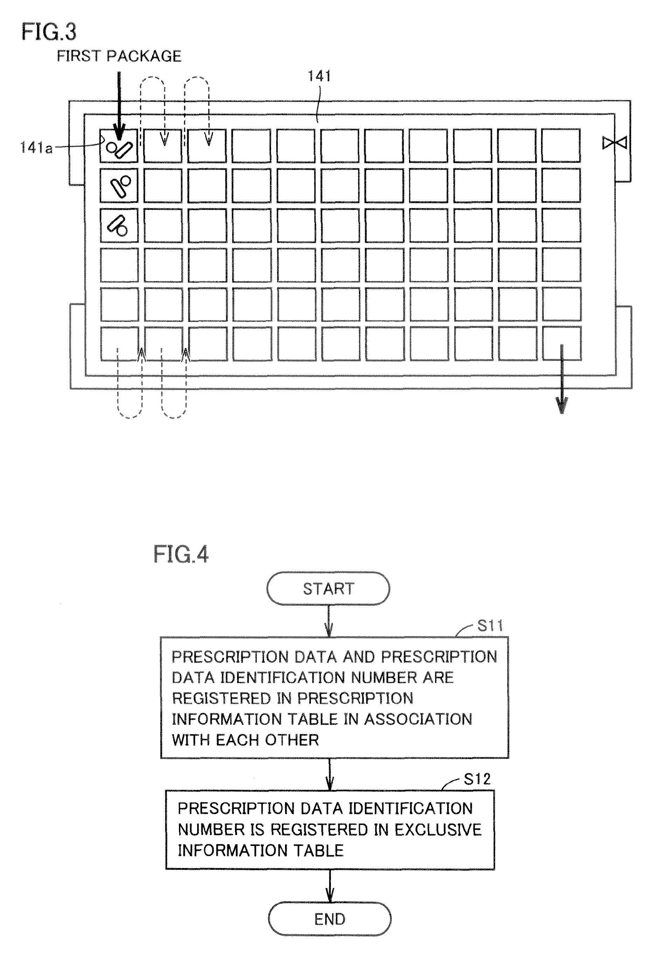

Fig. 3 is a plan view of the tray attached to the packaging machine shown in Fig. 2. Referring to Fig. 3, in tray 141 of the manually prepared drug supplying unit, the plurality of compartments 141 a are formed in the form of a lattice. Each compartment 141a is given a compartment number. Drugs are manually contained in compartments 141a.

Fig. 4 is a flowchart for illustrating an operation regarding preparation of prescription data in the host computer. Host computer 10 is provided with a database. The database has a prescription information table and an exclusive information table. In the prescription information table, prescription data and a prescription data identification number are stored in association with each other. In the exclusive information table, the prescription data identification number is stored.

When prescription data is input, prescription data regarding a plurality of patients belonging to the same group is created as one file, thus starting an operation of preparing the prescription data. When the operation of preparing the prescription data is started, in a step S11, the prescription data and the prescription data identification number are registered in the prescription information table in association with each other. Next, in a step S12, the same prescription data identification number as the prescription data identification number registered in step S11 is registered in the exclusive information table. In this way, the operation of preparing the prescription data is ended.

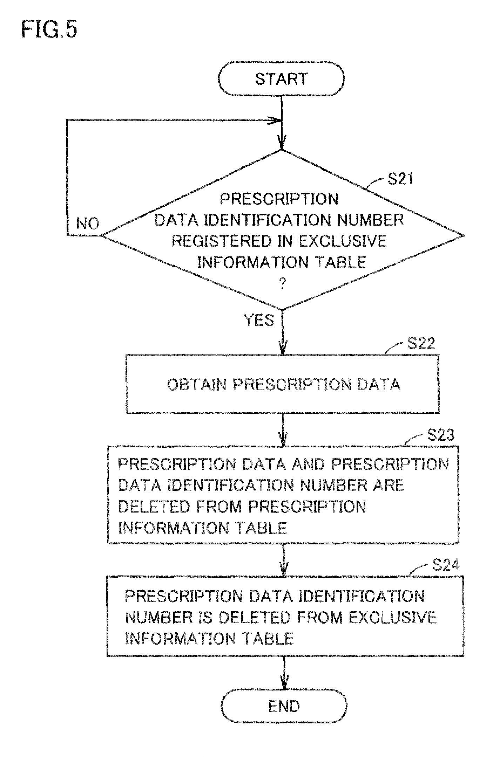

Fig. 5 is a flowchart for illustrating an operation regarding obtainment of the prescription data in the computer. When the operation of obtaining the prescription data is started, in a step S21, it is determined whether or not the prescription data identification number has been registered in the exclusive information table of the database of host computer 10. Until the prescription data identification number is registered in the exclusive information table, the determination in step S21 is repeated. When the prescription data identification number is registered in the exclusive information table, the process proceeds to a step S22.

In step S22, the prescription data identification number is obtained from the exclusive information table, and then the prescription data associated with the same prescription data identification number as this prescription data identification number is obtained from the prescription information table. Next, in a step S23, the prescription data obtained in step S22 and the prescription data identification number associated with this prescription data are deleted from the prescription information table. Next, in a step S24, the same prescription data identification number as the prescription data identification number deleted in step S23 is deleted from the exclusive information table. In this way, the operation of obtaining the prescription data is ended.

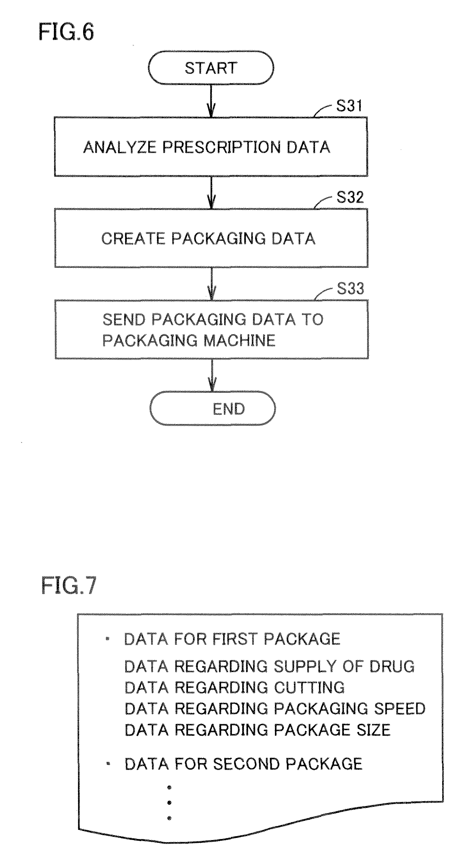

Fig. 6 is a flowchart for illustrating an operation regarding creation of the packaging data in the computer. When the prescription data regarding the plurality of patients belonging to the same group is obtained from host computer 10, the operation of creating the packaging data is started. When the operation of creating the packaging data is started, in a step S31, the obtained prescription data is analyzed. Next, in a step S32, based on the result of analysis, the packaging data is created. In doing so, one piece of packaging data is created for the prescription data regarding the plurality of patients belonging to the same group. Next, in a step S33, the packaging data created in step S32 is sent to packaging machine 100, and thereafter, the operation of creating the packaging data is ended.

Fig. 7 illustrates the packaging data. The packaging data is an aggregate of pieces of data each for one package. Each piece of data for one package include: data regarding supply of drug; data regarding cutting; data regarding packaging speed; and data regarding package size.

The data regarding supply of drug is data for supplying drugs from cassette drug supplying unit 101 and manually prepared drug supplying unit 102. The data regarding supply of drug designates supply of a drug from one of cassette drug supplying unit 101 and manually prepared drug supplying unit 102. In the case where the drug is supplied from cassette drug supplying unit 101, the data regarding supply of drug designates cassette 111 from which the drug is to be supplied, and designates a quantity of the drug to be supplied from cassette 111.

The data regarding cutting indicates whether to cut the package paper by cutting unit 152. For the final package, the data regarding cutting is set to perform cutting. For packages other than the final package, the data regarding cutting is set not to perform cutting. For the packages other than the final package, the data regarding cutting may be set appropriately to perform cutting.

The data regarding packaging speed is set based on the location of a cassette 111 having a drug contained therein. As a distance between cassette 111 and hopper 149 is longer, the packaging speed is made slower because it takes a longer time for the drug to be moved from cassette 111 to hopper 149. For example, in cassette drug supplying unit 101 of Fig. 2, the packaging speed for packaging a drug contained in a cassette 111 disposed at an upper side is made slower than the packaging speed for packaging a drug contained in a cassette 111 disposed at a lower side. By setting the packaging speed for each package, the packaging speed can be improved as a whole.

The data regarding package size is set based on the quantity of a drug. As the quantity of the drug is larger, the package size is made larger. The package size corresponds to the size of the package bag. By setting the package size for each package, waste of the package paper can be reduced as a whole.

Based on such packaging data, packaging machine 100 packages drugs. Packaging machine 100 sequentially performs the operation for the first package to the final package based on the pieces of data each for one package, thereby forming a series of package bags. After forming the package bag of the final package, packaging machine 100 sends the package bag of the final package to downstream relative to cutting unit 152. Then, the package paper is cut by cutting unit 152.

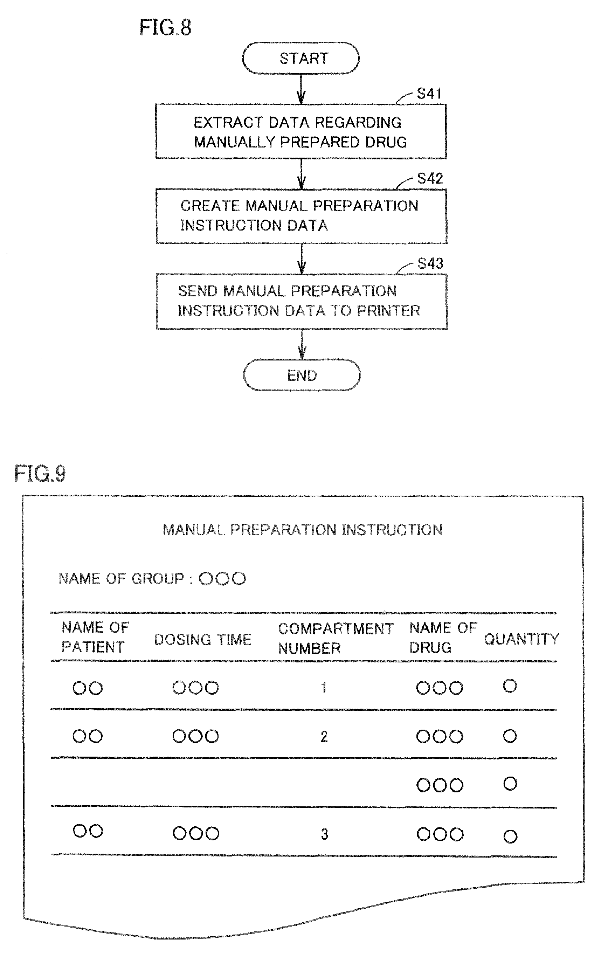

Fig. 8 is a flowchart for illustrating an operation regarding creation of the manual preparation instruction data in the computer. When prescription data regarding a plurality of patients belonging to the same group is obtained from host computer 10, the operation of creating manual preparation instruction data is started. When the operation of creating the manual preparation instruction data is started, in a step S41, data regarding a manually prepared drug to be supplied from manually prepared drug supplying unit 102 is extracted from the obtained prescription data. In doing so, with reference to a drug master database, it is determined whether or not the data regarding the drug in the prescription data is the data regarding the manually prepared drug.

Next, in a step S42, the extracted data regarding the manually prepared drug is associated with a compartment number so as to create manual preparation instruction data designating a compartment 141a, which is to contain the manually prepared drug, of tray 141. Next, in a step S43, the manual preparation instruction data created in step S42 is sent to printer 30, and then the operation of creating the manual preparation instruction data is ended.

Fig. 9 shows contents of the manual preparation instruction. The contents of the manual preparation instruction include the name of a group, the name of patients, dosing times, compartment numbers, the names of drugs, and the quantities thereof. The compartment numbers are associated with the names of the drugs and the quantities thereof, thereby indicating the names and quantities of the manually prepared drugs to be contained in compartments 141a of tray 141. The compartment numbers are further associated with the names of the patients and the dosing times. In accordance with such a manual preparation instruction, an operator can contain, in compartments 141 a of tray 141, the manually prepared drugs for the plurality of patients at one time.

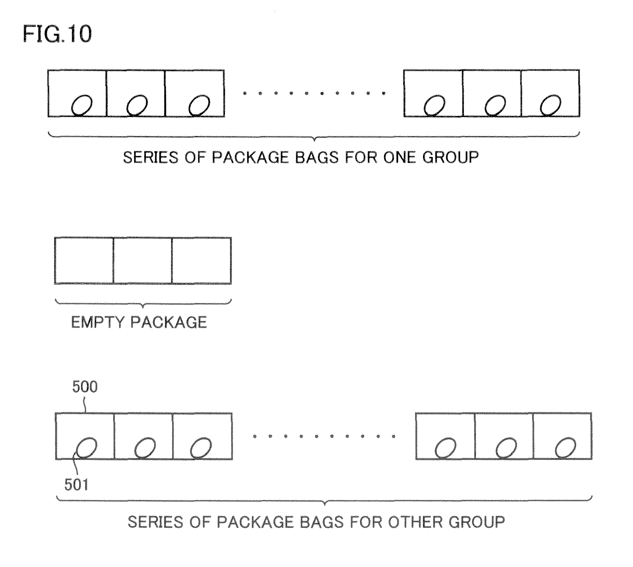

Fig. 10 illustrates a result of packaging. An empty package having no drug contained therein is formed between a series of package bags for one group and a series of package bags for the other group. However, an empty package having no drug contained therein is not formed between package bags for different patients of the same group, thereby reducing waste of the package paper.

In the above-described first embodiment, host computer 10 is provided with the database via which computer 20 obtains the prescription data, but in another embodiment, the database may be provided in computer 20. Further, the database may be provided in a device other than host computer 10 and computer 20. Further, the prescription data may be transferred from host computer 10 to computer 20 without using such a database.

Further, in the first embodiment, in host computer 10, prescription data regarding a plurality of patients is collectively handled based on a group as a unit. However, in another embodiment, in computer 20, the prescription data regarding the plurality of patients may be collectively handled based on a group as a unit. In this case, computer 20 may sequentially obtain prescription data regarding each patient from host computer 10. Alternatively, computer 20 may obtain it by an operator manually inputting the prescription data into computer 20.

Further, in the first embodiment, printer 30 serves as the manual preparation instruction data output unit, but the manual preparation instruction data output unit is not limited to printer 30. A display unit provided in computer 20 may display a manual preparation instruction screen. Further, the manual preparation instruction may be printed using a journal printer provided in packaging machine 100, or a manual preparation instruction screen may be displayed by a display unit provided in packaging machine 100.

The embodiments disclosed herein are illustrative and non-restrictive in any respect. The scope of the present invention is defined by the terms of the claims, rather than the embodiments described above, and is intended to include any modifications within the scope and meaning equivalent to the terms of the claims.

### REFERENCE SIGNS LIST

1: drug preparing system; 10: host computer; 20: computer; 30: printer; 100: packaging machine; 101: cassette drug supplying unit; 102: manually prepared drug supplying unit; 111: cassette; 141: tray; 141a: compartment; 149: hopper; 150: printing unit; 152: cutting unit; 153: transporting unit; 154: sealing unit; 156: package paper roll supporting unit.

## Claims

1. A drug packaging device comprising:
a packaging data creating unit (20) that creates one piece of packaging data based on prescription data regarding a plurality of patients belonging to a same group; and
a drug packaging unit (100) that packages a drug based on the packaging data created by said packaging data creating unit.

2. The drug packaging device according to claim 1, wherein said packaging data creating unit obtains the prescription data regarding the plurality of patients belonging to the same group, from a host computer (10) as one file.

3. The drug packaging device according to claim 1, wherein said packaging data is an aggregate of pieces of data each for one package, and each of said pieces of data each for one package includes data regarding a packaging speed.

4. The drug packaging device according to claim 1, wherein said packaging data is an aggregate of pieces of data each for one package, and each of said pieces of data each for one package includes data regarding a package size.

5. The drug packaging device according to claim 1, wherein
said drug packaging unit has a manually prepared drug supplying unit (102) that sequentially supplies drugs contained in a plurality of compartments formed in a tray, and is configured to package the drugs supplied from said manually prepared drug supplying unit,
the drug packaging device further comprising:
a manual preparation instruction data creating unit (20) that extracts, from the prescription data regarding the plurality of patients belonging to the same group, data regarding a manually prepared drug to be supplied from said manually prepared drug supplying unit, and that creates manual preparation instruction data designating a compartment, which is to contain the manually prepared drug, of the tray; and
a manual preparation instruction data output unit (30) that outputs the manual preparation instruction data created by said manual preparation instruction data creating unit.
